(19) Europäisches Patentamt
European Patent Office
Office européen des brevets

(11) **EP 2 990 847 A1**

(12) **EUROPEAN PATENT APPLICATION**
published in accordance with Art. 153(4) EPC

(43) Date of publication:
**02.03.2016 Bulletin 2016/09**

(51) Int Cl.:
***G02B 13/04*** (2006.01)   ***A61B 1/00*** (2006.01)
***G02B 13/18*** (2006.01)

(21) Application number: **14787932.4**

(22) Date of filing: **03.04.2014**

(86) International application number:
**PCT/JP2014/059896**

(87) International publication number:
**WO 2014/175038 (30.10.2014 Gazette 2014/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **22.04.2013   JP 2013089431**

(71) Applicant: **OLYMPUS CORPORATION**
**Shibuya-ku**
**Tokyo 151-0072 (JP)**

(72) Inventor: **NAKAMURA, Minoru**
**Shibuya-ku, Tokyo 151-0072 (JP)**

(74) Representative: **Schicker, Silvia**
**Wuesthoff & Wuesthoff**
**Patentanwälte PartG mbB**
**Schweigerstraße 2**
**81541 München (DE)**

(54) **WIDE-ANGLE OBJECTIVE OPTICAL SYSTEM**

(57)    Excellent aberration correction, suitability for high-definition and high-pixel solid state imaging elements, a small outer diameter of an end and reduction in manufacturing cost are achieved.

A wide-angle objective optical system includes, in order from an object side to an image side: a first lens group (G1); and a second lens group (G2) having positive refractive power. The first lens group includes, in order from the object side to the image side: a first lens (L1) having negative refractive power; a second lens (L2) having a concave face as an object-side face and having negative refractive power; a brightness diaphragm (S); and a third lens (L3) having positive refractive power. The following condition expressions are satisfied:

$$-0.5 < f/f1 < 0.5 \qquad (1)$$

$$0.3 < f/f2 < 0.7 \qquad (2)$$

wherein f1, f2 and f are focal distances of the first lens group, the second lens group, and the entire wide-angle objective optical system, respectively.

**EP 2 990 847 A1**

**(Cont. next page)**

# FIG. 1

**Description**

{Technical Field}

The present invention relates to a wide-angle objective optical system that is compact and applicable to endoscopes and monitoring cameras, and more particularly, to a wide-angle objective optical system that has a 100 degree or more angle of view.

{Background Art}

Definition enhancement and increase in pixel value for solid state imaging elements such as CCD or CMOS sensors have been promoted recently. On the other hand, for objective lenses applicable to endoscopes and monitoring cameras, wider photographing ranges, that is, wider angles of view have been conventionally demanded. Further, since a channel for inserting illuminating optical systems and treatment instruments and an air/water supply nozzle need to be arranged at a distal end of an endoscope, reducing diameters of ends of objective lenses has been demanded. Also for monitoring cameras, reducing diameters of ends of lenses has been demanded for making the lenses inconspicuous.
As such a wide-angle objective optical system, for example, each of PTL 1 to PTL 3 discloses a wide-angle objective optical system including, in order from an object side, a first lens having negative refractive index, a second lens having negative refractive index, a brightness diaphragm and a third lens having positive refractive index. To achieve a smaller outer diameter of an end as well as a wider angle, in each of the objective optical systems, the first lens has negative refractive index and the brightness diaphragm is arranged as near an object as possible so that an entrance pupil is placed at a front side.
For such objective optical systems, reduction in manufacturing cost has also been demanded. In mobile phones or the like, lenses are manufactured in large amounts in a short time by an injection molding technique using a plastic optical material to bring about cost down.
To achieve definition enhancement and increase in pixel value of a solid state imaging element, an aberration generated in an objective optical system needs to be further reduced. To achieve a wider angle, a chromatic aberration of magnification, which has a great influence on peripheral performance, particularly, needs to be reduced.

{Citation List}

{Patent Literature}

{PTL 1} Japanese Unexamined Patent Application, Publication No. Hei 10-20189
{PTL 2} Japanese Unexamined Patent Application, Publication No. Hei 10-197787
{PTL 3} Japanese Unexamined Patent Application, Publication No. Sho 61-162021

{Summary of Invention}

{Technical Problem}

However, in the objective optical system of PTL 1, since refractive power arrangement in each lens causes reduction in the overall lens length, a large amount of glass materials having a high refractive index needs to be used. Thus, the manufacturing cost increases. In the objective optical system of PTL 2, the number of the included lenses is excessively small. Thus, imaging performance is insufficient for definition enhancement and a wider angle of view of a solid state imaging element. In the objective optical system of PTL 3, refractive power arrangement and shapes of the first lens and the second lens prevent the position of the entrance pupil from being placed at the front side. Thus, the outer diameter of the end becomes large.
The present invention has been made in view of the aforementioned situations. The object of the present invention is to provide a wide-angle objective optical system that enables excellent aberration correction, suitability for high-definition and high-pixel solid state imaging elements, a small outer diameter of an end and reduction in manufacturing cost.

{Solution to Problem}

To achieve the aforementioned object, the present invention provides the following solutions.

An aspect of the present invention is a wide-angle objective optical system comprising, in order from an object side to an image side: a first lens group; and a second lens group having positive refractive power, wherein the first lens group comprises, in order from the object side to the image side: a first lens having negative refractive power; a second lens having a concave face as an object-side face and having negative refractive power; a brightness diaphragm; and a third lens having positive refractive power, and the following condition expressions are satisfied:

$$-0.5 < f/f1 < 0.5 \qquad (1)$$

$$0.3 < f/f2 < 0.7 \qquad (2)$$

wherein f1 is a focal distance of the first lens group; f2 is a focal distance of the second lens group; and f is a focal distance of the entire wide-angle objective optical system.

In the present aspect, since the wide-angle objective optical system has two separate lens groups: the first lens group and the second lens group having positive refractive power, aberrations of the respective lens groups can be corrected independently and loads of the aberration correction can be distributed between the two lens groups in a well-balanced manner. Consequently, a high-performance objective optical system can be obtained with the smaller number of the lenses.

Further, since the first lens group comprises, in order from the object side to the image side: the first lens having negative refractive power; the second lens having a concave face as the object-side face and having negative refractive power; the brightness diaphragm; and the third lens having positive refractive power, a position of an entrance pupil can be placed as near an object as possible. Thus, an outer diameter of an end of the objective optical system can be reduced.

In this way, the present aspect enables excellent aberration correction, suitability for high-definition and high-pixel solid state imaging elements, and further, a small outer diameter of the end, and reduction in manufacturing cost.

Furthermore, in the aforementioned aspect, it is preferable that the following condition expression is satisfied:

$$0.8 < D/f < 1.2 \qquad (3)$$

wherein D is an air space between a curved face having refractive power nearest to an image of the first lens group and a curved face having refractive power nearest to an object of the second lens group.

By this configuration, an off-axis aberration can be appropriately corrected, and further, the diameter of the end can be reduced.

Moreover, in the aforementioned aspect, it is preferable that the second lens group includes, in order from the object side to the image side, a fourth lens having positive refractive power, a fifth lens having negative refractive power and a sixth lens having positive refractive power.

By this configuration, a color of magnification, an astigmatic difference and a field curvature can be appropriately corrected with the small number of the included lenses and an optical member having a low refractive index. Thus, while high performance is achieved, the manufacturing cost can be reduced.

Furthermore, in the aforementioned aspect, it is preferable that the second lens group includes, in order from the object side to the image side, a fourth lens having positive refractive power, a fifth lens having negative refractive power, a sixth lens having positive refractive power and a seventh lens having positive refractive power.

By this configuration, a color of magnification, an astigmatic difference and a field curvature can be appropriately corrected with an optical member having a low refractive index. Thus, while high performance is achieved, the manufacturing cost can be reduced.

Moreover, in the aforementioned aspect, it is preferable the following condition expression is satisfied:

$$0.5 < f/f13 < 0.9 \qquad (4)$$

wherein f13 is a focal distance of the third lens having positive refractive power of the first lens group.

By this configuration, an off-axis aberration such as a field curvature, an astigmatic difference, a coma aberration and a chromatic aberration of magnification can be appropriately corrected.

Furthermore, in the aforementioned aspect, it is preferable that the following condition expression is satisfied:

$$-0.3 < f/f12 < 0.0 \qquad (5)$$

wherein f12 is a focal distance of the second lens having negative refractive power of the first lens group.

By this configuration, an off-axis aberration such as a field curvature, an astigmatic difference, a coma aberration and a chromatic aberration of magnification can be appropriately corrected.

{Advantageous Effects of Invention}

The present invention provides an effect that enables excellent aberration correction, suitability for high-definition and high-pixel solid state imaging elements, and further, a small outer diameter of an end and reduction in manufacturing cost.

{Brief Description of Drawings}

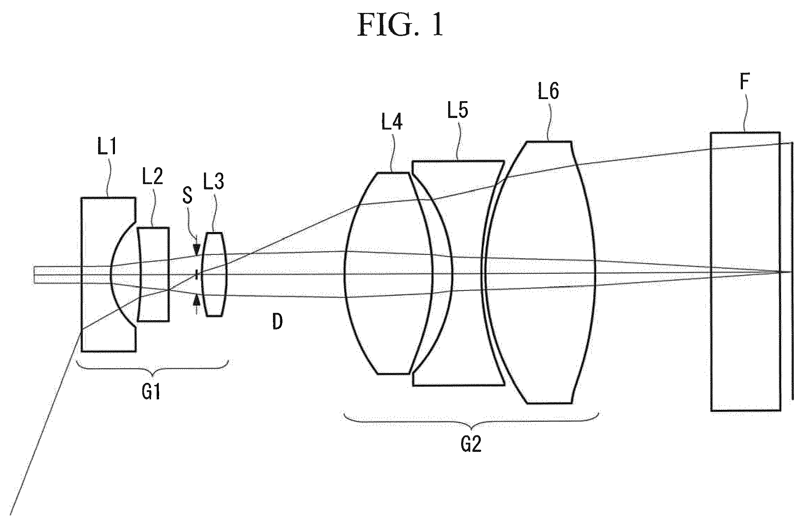

{Fig. 1} Fig. 1 is a sectional view of an overall configuration of a wide-angle objective optical system of an embodiment of the present invention.

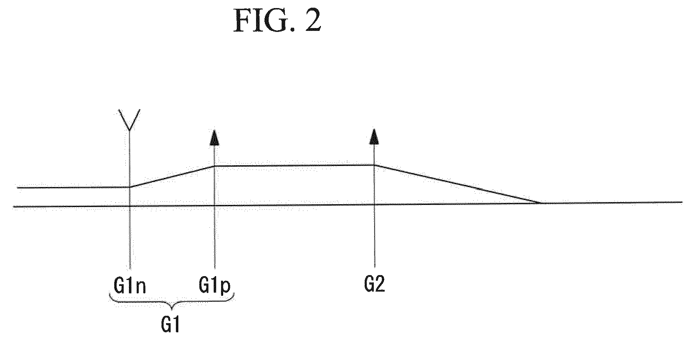

{Fig. 2} Fig. 2 is an explanatory diagram of an afocal arrangement.

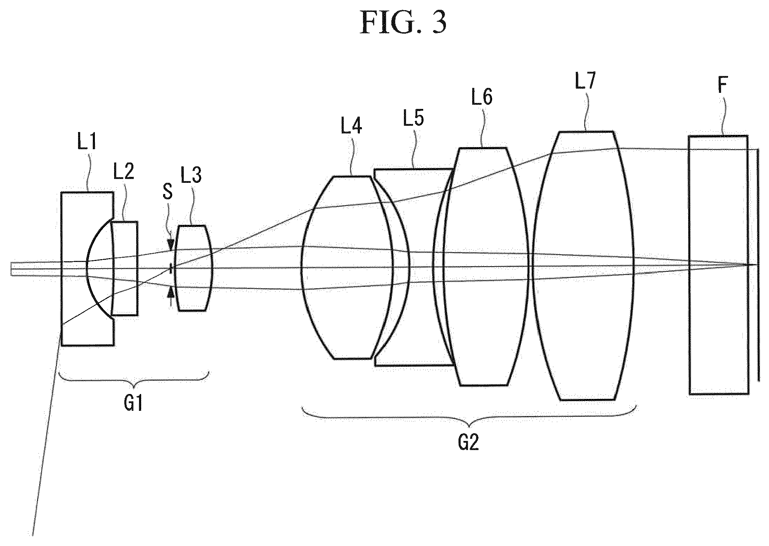

{Fig. 3} Fig. 3 is a sectional view of an overall configuration of a wide-angle objective optical system in another example of the embodiment of the present invention.

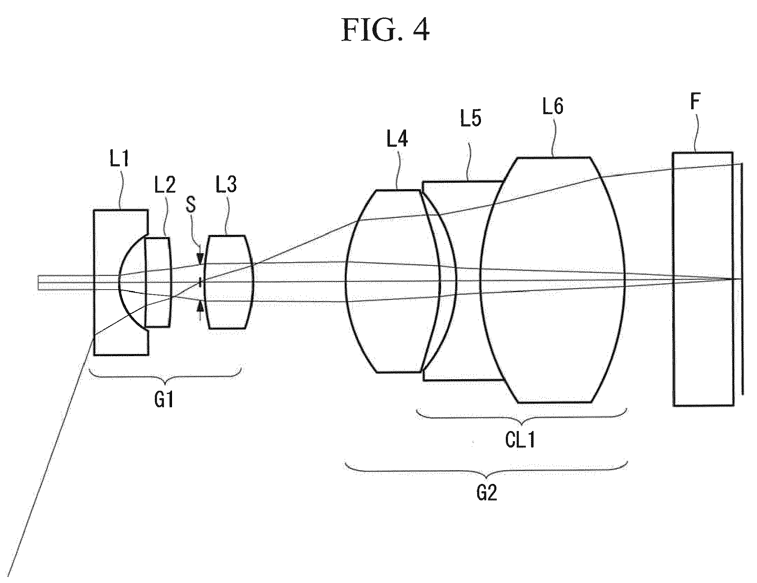

{Fig. 4} Fig. 4 is a sectional view of an overall configuration of a wide-angle objective optical system in still another example of the embodiment of the present invention.

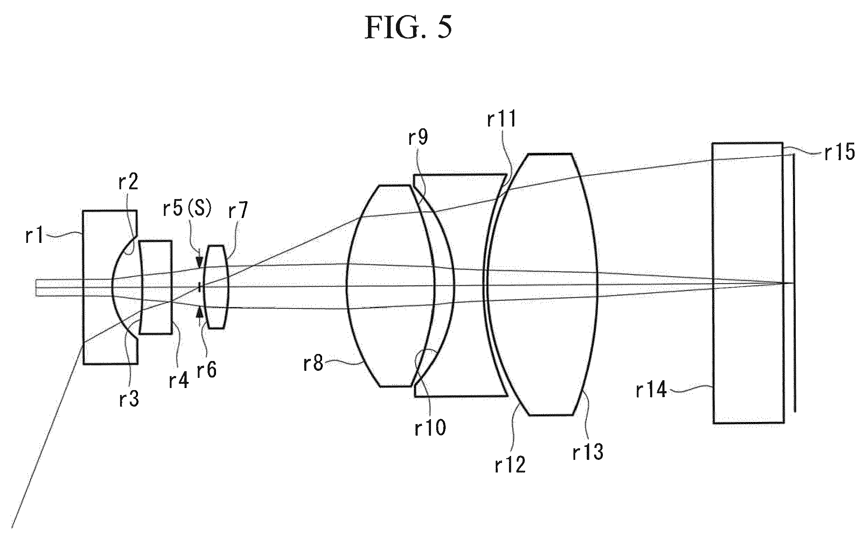

{Fig. 5} Fig. 5 is a sectional view of an overall configuration of a wide-angle objective optical system of Example 1 of the present invention.

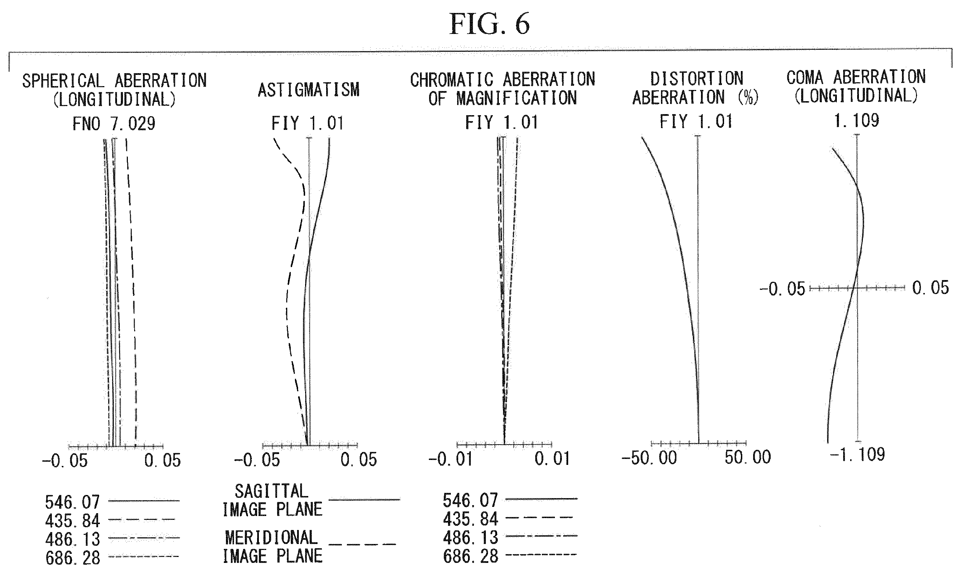

{Fig. 6} Fig. 6 is a diagram of aberration curves of the wide-angle objective optical system of Fig. 5.

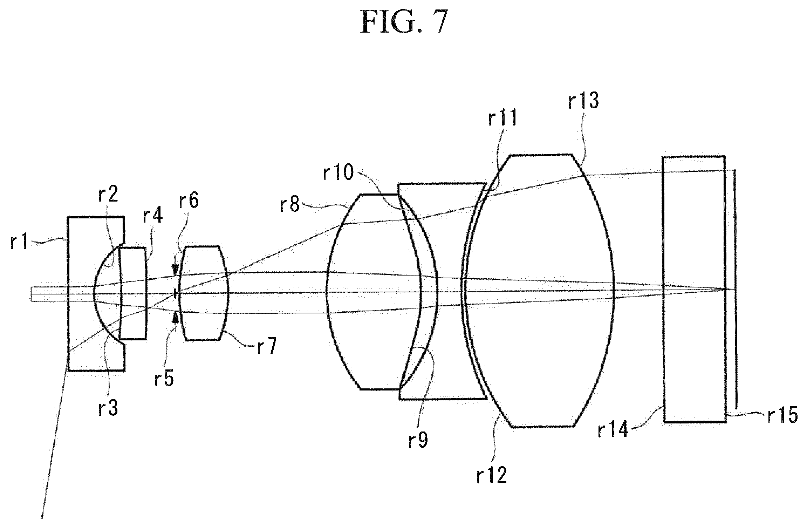

{Fig. 7} Fig. 7 is a sectional view of an overall configuration of a wide-angle objective optical system of Example 2 of the present invention.

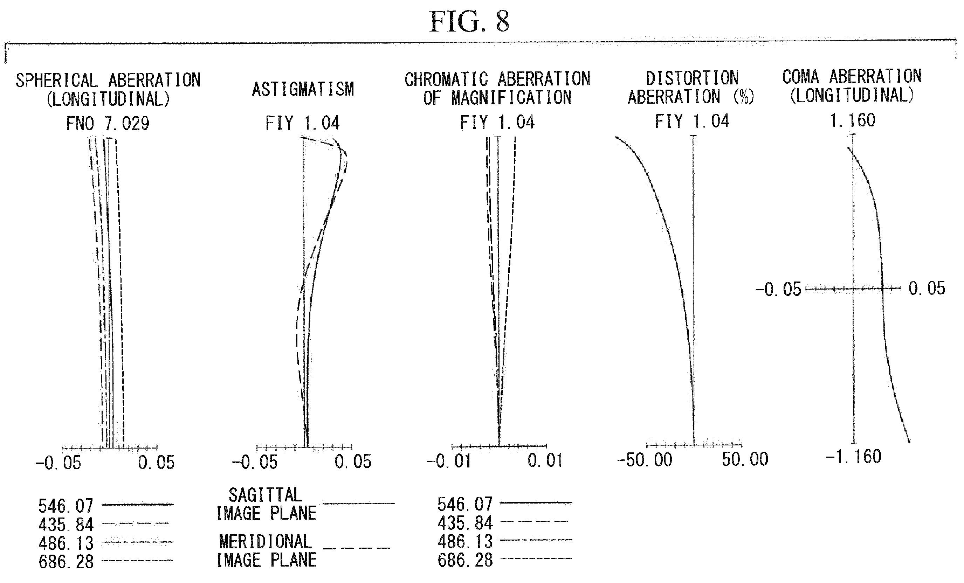

{Fig. 8} Fig. 8 is a diagram of aberration curves of the wide-angle objective optical system of Fig. 7.

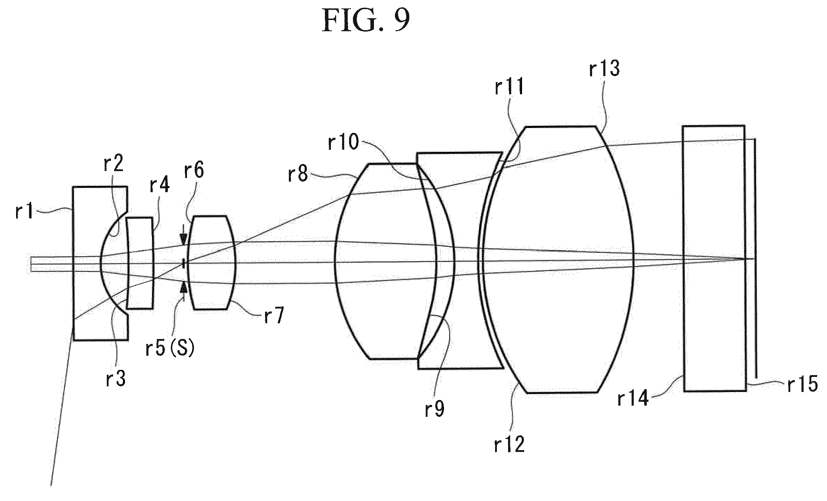

{Fig. 9} Fig. 9 is a sectional view of an overall configuration of a wide-angle objective optical system of Example 3 of the present invention.

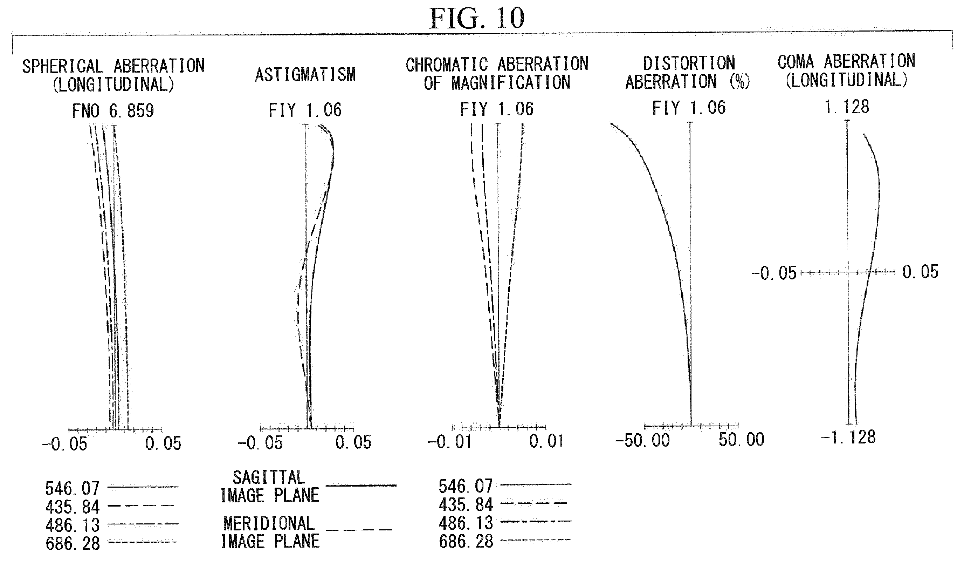

{Fig. 10} Fig. 10 is a diagram of aberration curves of the wide-angle objective optical system of Fig. 9.

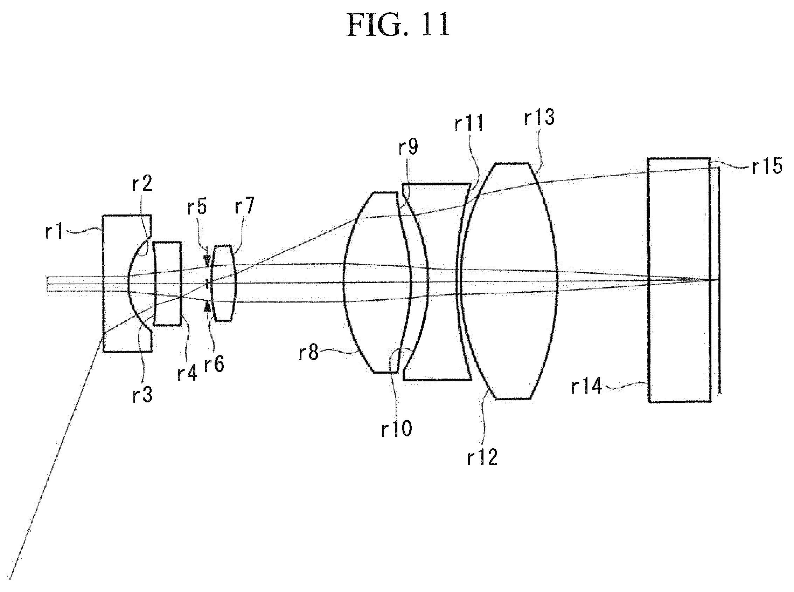

{Fig. 11} Fig. 11 is a sectional view of an overall configuration of a wide-angle objective optical system of Example 4 of the present invention.

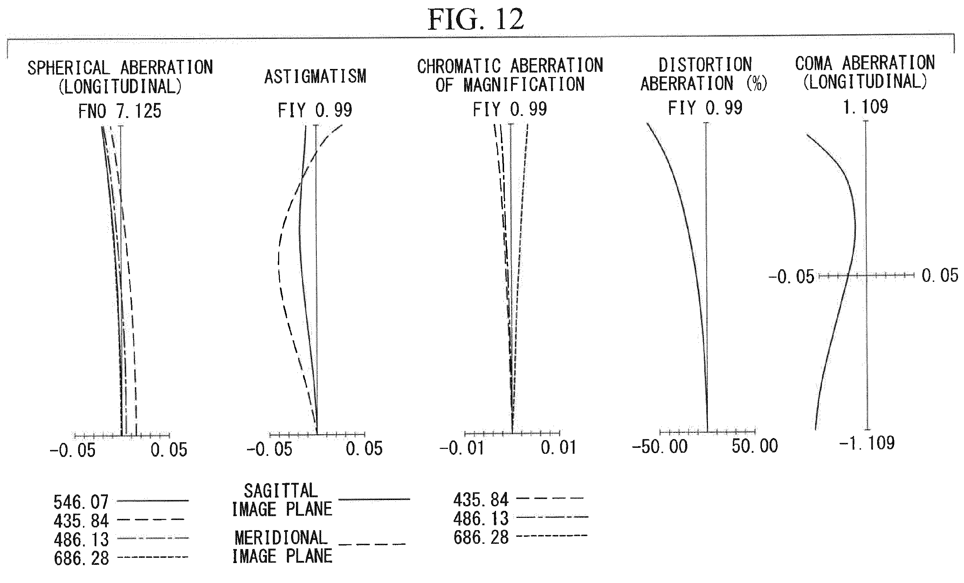

{Fig. 12} Fig. 12 is a diagram of aberration curves of the wide-angle objective optical system of Fig. 11.

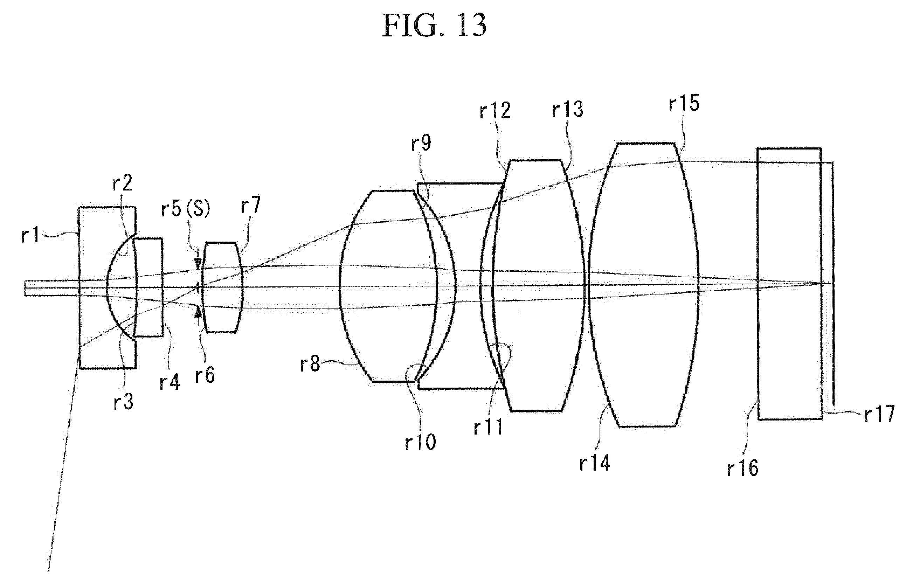

{Fig. 13} Fig. 13 is a sectional view of an overall configuration of a wide-angle objective optical system of Example 5 of the present invention.

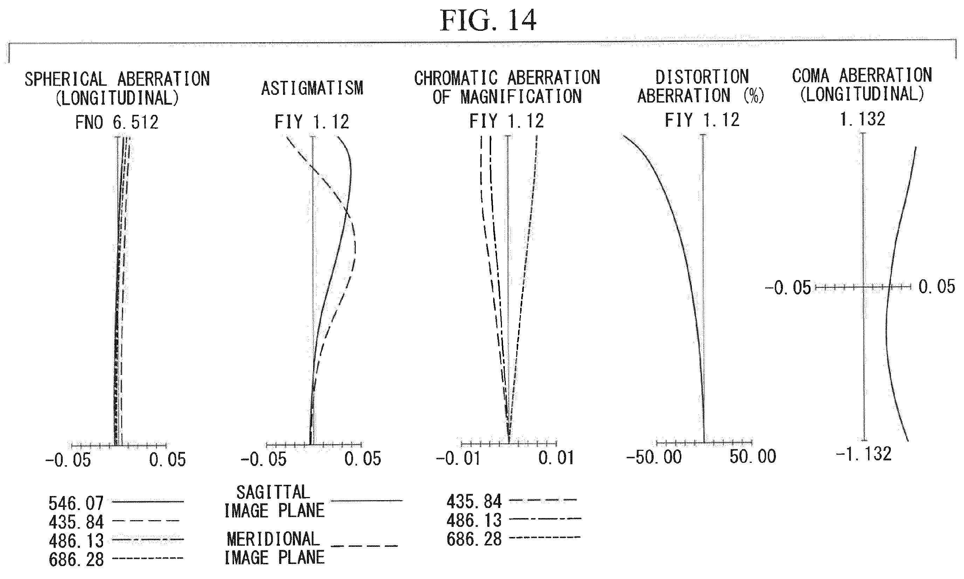

{Fig. 14} Fig. 14 is a diagram of aberration curves of the wide-angle objective optical system of Fig. 13.

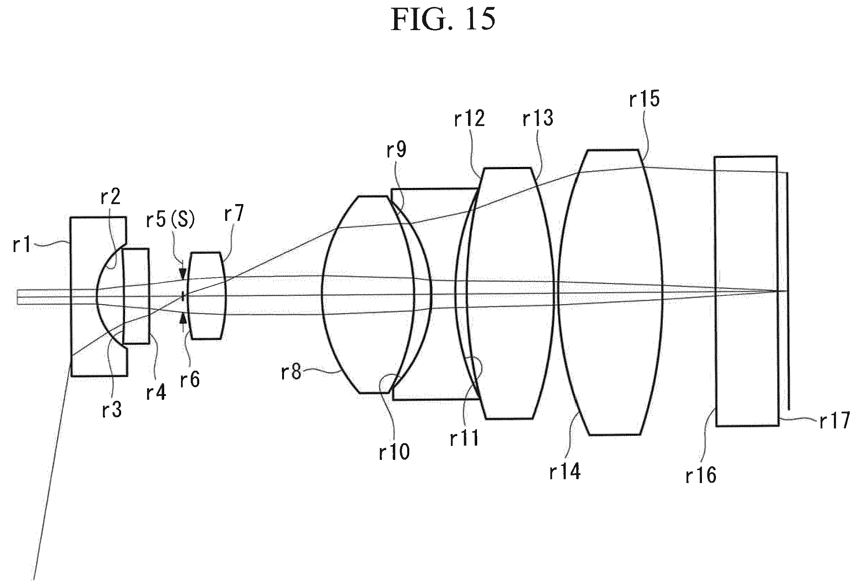

{Fig. 15} Fig. 15 is a sectional view of an overall configuration of a wide-angle objective optical system of Example 6 of the present invention.

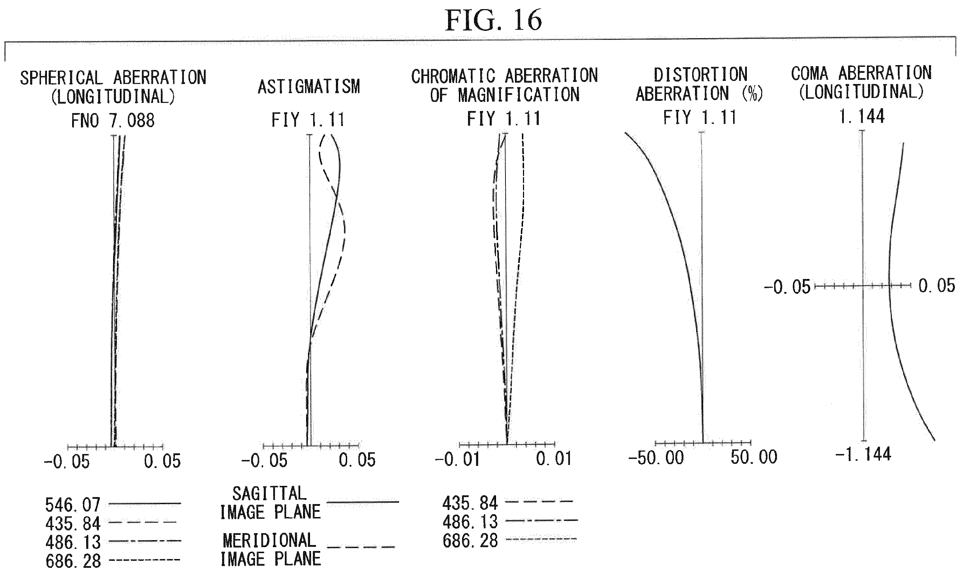

{Fig. 16} Fig. 16 is a diagram of aberration curves of the wide-angle objective optical system of Fig. 15.

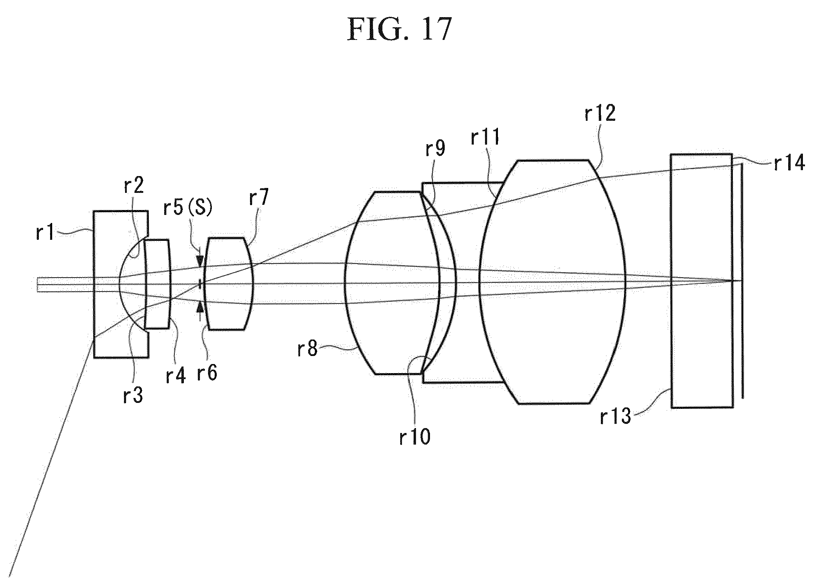

{Fig. 17} Fig. 17 is a sectional view of an overall configuration of a wide-angle objective optical system of Example 7 of the present invention.

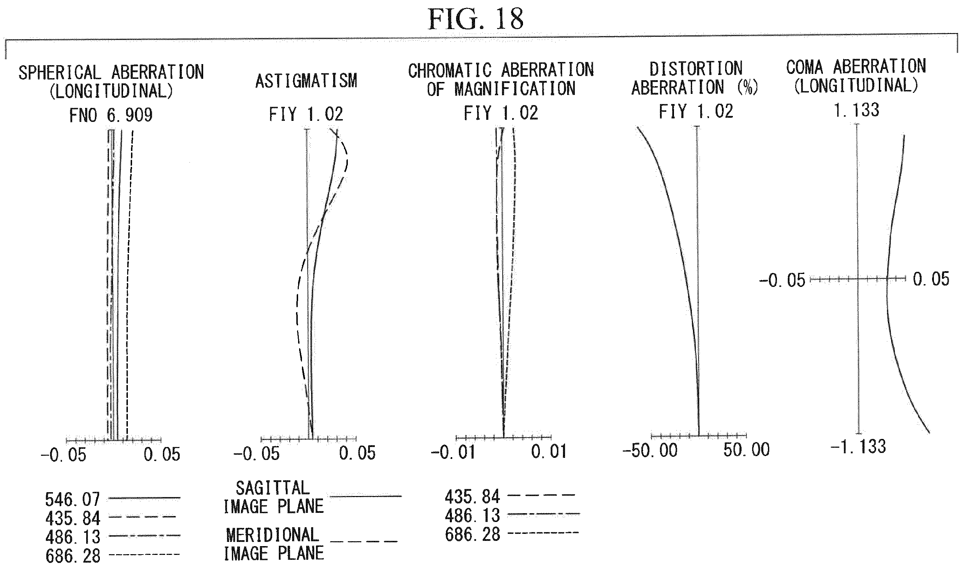

{Fig. 18} Fig. 18 is a diagram of aberration curves of the wide-angle objective optical system of Fig. 17.

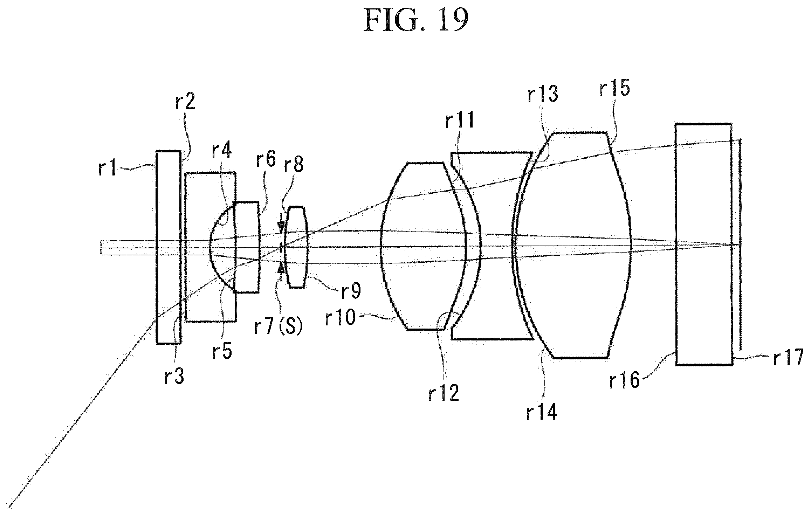

{Fig. 19} Fig. 19 is a sectional view of an overall configuration of a wide-angle objective optical system of Example 8 of the present invention.

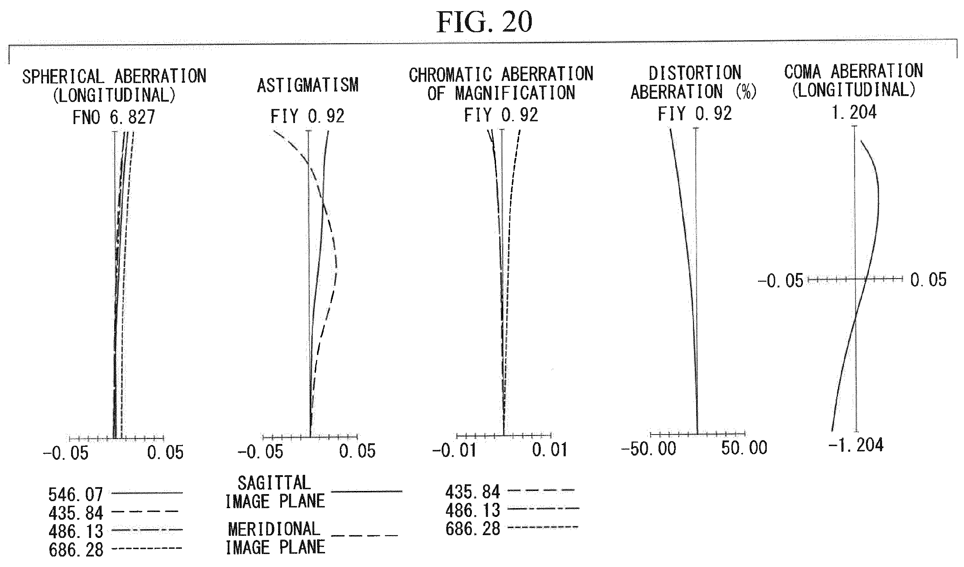

{Fig. 20} Fig. 20 is a diagram of aberration curves of the wide-angle objective optical system of Fig. 19.

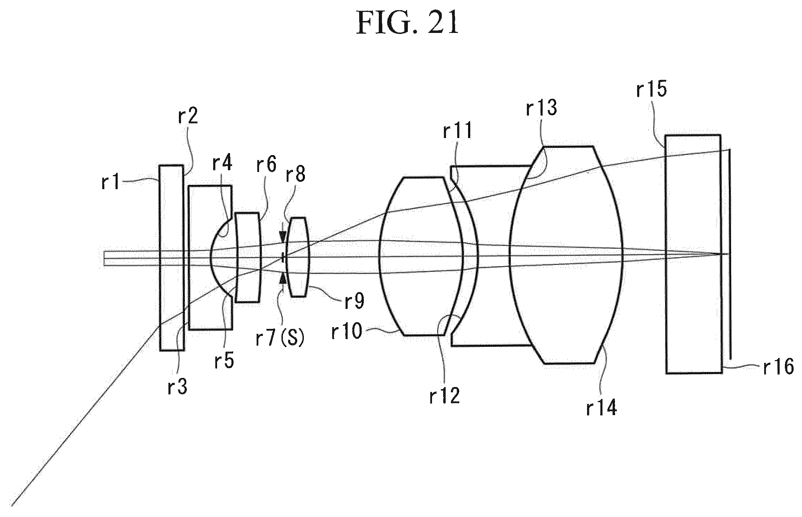

{Fig. 21} Fig. 21 is a sectional view of an overall configuration of a wide-angle objective optical system of Example 9 of the present invention.

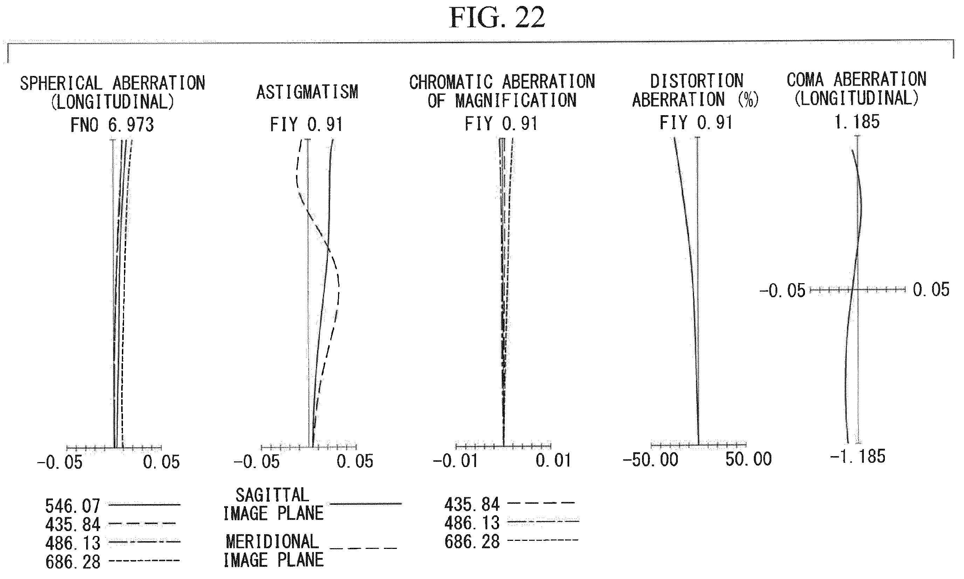

{Fig. 22} Fig. 22 is a diagram of aberration curves of the wide-angle objective optical system of Fig. 21.

{Description of Embodiments}

Hereinafter, descriptions will be given of a wide-angle objective optical system of an embodiment of the present invention with reference to the drawings.

Fig. 1 illustrates a sectional view of an overall configuration of the wide-angle objective optical system of the present embodiment. As illustrated in Fig. 1, the wide-angle objective optical system includes, in order from an object side to an image side, a first lens group G1 and a second lens group G2 having positive refractive power (hereinafter referred to as "positive" simply).

The first lens group G1 includes, in order from the object side to the image side, a first lens L1 having negative refractive power (hereinafter referred to as "negative" simply), a negative second lens L2 having a concave face as an object-side face, a brightness diaphragm S and a positive third lens L3.

The second lens group G2 includes, in order from the object side, a positive fourth lens L4, a negative fifth lens L5 and a positive sixth lens L6.

An image pickup element (not illustrated) is arranged in the vicinity of an image plane of the wide-angle objective optical system. The image pickup element is included in the wide-angle objective optical system and an image pickup optical system. To the image pickup element, a parallel plate F that protects an imaging area is attached.

The wide-angle objective optical system is divided into two groups: the first lens group G1 and the second lens group G2 having positive refractive power, as in the present embodiment, and the first lens group is further divided into a negative front group G1n and a positive rear group G1p in an afocal arrangement. Consequently, aberrations of the wide-angle objective optical system are corrected with high performance and the number of the included lenses is reduced. Thus, such a low refractive index can be achieved that allows usage of a plastic optical material, and a wide angle can also be achieved.

Afocal arrangement herein means, as illustrated in Fig. 2, that a focal distance f2 of the second lens group, a focal distance fg1n and a focal distance fg1p of the front group and the rear group, respectively, included in the first lens group G1 and a principal point interval Dnp between G1n and G1p substantially satisfy the following expression (A):

$$\mathrm{Dnp} \approx \mathrm{fg1p} + \mathrm{fg1n} \qquad (A).$$

At that time, an entire focal distance f approximates the following expression (B):

$$\mathrm{f} \approx -\mathrm{fg1n/fg1p} \times \mathrm{f2} \qquad (B),$$

wherein f2 is a focal distance of the second lens group G2.

The value of "-fg1n/fg1p" is referred to as an afocal magnification.

Such afocal arrangement enables correction of aberrations of the first lens group G1 and the second lens group G2 separately. Further, loads of the aberration correction can be distributed between the first lens group G1 and the second lens group G2 in a well-balanced manner. Consequently, a high-performance objective optical system can be achieved with the small number of the lenses.

The wide-angle objective optical system satisfies the following condition expressions (1) and (2).

$$-0.5 < \mathrm{f/f1} < 0.5 \qquad (1)$$

$$0.3 < \mathrm{f/f2} < 0.7 \qquad (2)$$

wherein f1 is a focal distance of the first lens group G1; f2 is a focal distance of the second lens group G2; and f is a focal distance of the entire wide-angle objective optical system.

The condition expression (1) is a condition to achieve an afocal arrangement. If f/f1 is equal to or lower than the lower limit of -0.5, stronger positive refractive power is required for the second lens group G2, which makes the aberration correction difficult. If f/f1 is equal to or higher than the upper limit of 0.5, the load of the aberration correction in the first lens group increases, which deteriorates the performance.

The condition expression (2) defines the refractive power of the second lens group G2. If f/f2 is equal to or lower than the lower limit of 0.3, the smaller afocal magnification is required for the first lens group G1, which increases the load of the aberration correction in the first lens group G1, and further, as the focal distance of the second lens group G2 increases, the overall length increases. If f/f2 is equal to or higher than the upper limit of 0.7, the load of the aberration correction in the second lens group G2 becomes excessively large, which deteriorates the performance.

To make an outer diameter of the end small, a position of an entrance pupil needs to be placed as near an object as possible. Thus, the first lens group G1 is configured by the negative first lens L1, the negative second lens L2 having a concave face as the object-side face, the brightness diaphragm S and the positive third lens L3, in order from the object side. Placing the brightness diaphragm S before the positive third lens L3 makes the position of the entrance pupil as near the object as possible.

Moreover, the stronger refractive power of the third lens L3 enables a smaller space between the second lens L2 and the third lens L3 to place the position of the entrance pupil at the front side, with keeping the afocal magnification same. However, synthetic refractive power of the first lens L1 and the second lens L2 increases simultaneously. Thus, to correct an aberration, negative refractive power is distributed into the first lens L1 and the second lens L2.

Furthermore, the second lens L2 has a concave face as the object-side face and has negative refractive power. Thus, the rear-side principal point position of the synthetic refractive power of the first lens L1 and the second lens L2 can be placed forward, which allows the afocal magnification to be small.

The wide-angle objective optical system satisfies the following condition expression:

$$0.8 < D/f < 1.2 \qquad (3)$$

wherein D is an air space between a curved face having refractive power nearest to an image of the first lens group G1 and a curved face having refractive power nearest to an object of the second lens group G2.

In the condition expression (3), if D/f is equal to or lower than the lower limit of 0.8, an off-axis aberration (field curvature, astigmatic difference, coma aberration) generated in the first lens group G1 is difficult to be corrected by the second lens group G2 formed of glass having a low refractive index, which prevents achievement of a wider angle in the wide-angle objective optical system. The reason is that the narrower space makes an off-axis ray pass the second lens group G2 at a lower height, which prevents aberration correction independent of an on-axis ray.

Although strengthening the refractive power of the second lens group G2 and lowering the height of the ray relatively are conceivable, the second lens group G2 needs to use glass having a high refractive index. In contrast, if D/f is equal to or higher than the upper limit of 1.2, the distance from the second lens group G2 excessively increases, a height at which an off-axis ray enters the second lens group G2 becomes higher. As a result, the outer diameter of the second lens group G2 becomes large and an aberration is generated to bring about performance deterioration. The upper limit is further preferably a value lower than 1.0, at which the size and the performance are well-balanced.

If the lower limit is changed from 0.8 to 0.6 in the condition expression (3), the wide-angle objective optical system, including the first lens L1 of the first lens group G1, can be formed of a plastic optical material which has a low refractive index and is suitable for injection molding. The reason is that the afocal magnification of the first lens group G1 can be large and the height at which an off-axis ray passes the second lens group G2 can be held.

The wide-angle objective optical system satisfies the following condition expression:

$$0.5 < f/f13 < 0.9 \qquad (4)$$

wherein f13 is a focal distance of the third lens having positive refractive power of the first lens group.

If f/f13 is equal to or lower than the lower limit of 0.5 in the condition expression (4), the space between the second lens L2 and the third lens L3 of the first lens group G1 becomes wider in order to secure the afocal magnification. Thus, the outer diameter of the end becomes large. If f/f13 is equal to or higher than the upper limit of 0.9, the refractive power of the first lens L1 and the third lens L3 of the first lens group G1 is excessively strong. Thus, an off-axis aberration (field curvature, astigmatic difference, coma aberration, chromatic aberration of magnification) is difficult to be corrected.

The wide-angle objective optical system further satisfies the following condition expression:

$$-0.3 < f/f12 < 0.0 \qquad (5)$$

wherein f12 is a focal distance of the second lens having negative refractive power of the first lens group.

If f/f12 is equal to or lower than the lower limit of - 0.3 in the condition expression (5), the space between the second lens L2 and the third lens L3 of the first lens group G1 becomes wider in order to secure the afocal magnification. Thus, the outer diameter of the end becomes large. If f/f12 is equal to or higher than the upper limit of 0.0, the refractive power of the first lens L1 of the first lens group G1 is excessively strong. Thus, an off-axis aberration (field curvature, astigmatic difference, coma aberration, chromatic aberration of magnification) is difficult to be corrected.

In the present embodiment, as described above, the second lens group G2 is a triplet configuration including the positive fourth lens L4, the negative fifth lens L5 and the positive sixth lens L6, which are arranged in order from the object side. Accordingly, a color of magnification, an astigmatic difference and a field curvature can be appropriately corrected with the small number of the included lenses and the optical member having a low refractive index.

The second lens group G2 is not limited to the aforementioned configuration but, for example, may have a quadruplet configuration in which the fourth lens L4 having positive refractive power, the fifth lens L5 having negative refractive power, the sixth lens L6 having positive refractive power and a seventh lens L7 having positive refractive power are included, as illustrated in Fig. 3, that is, a lens having positive refractive power is added to a triplet. This configuration can reduce loads to the lenses having positive refractive power of the second lens group. Consequently, a color of magnification, an astigmatic difference and a field curvature can be appropriately corrected with the optical member having a low refractive index.

If, as illustrated in Fig. 4, the second lens group G2 includes a cemented lens CL1 that is formed by cementing the fifth lens 5 and the sixth lens 6, a color of magnification is corrected more appropriately although an angle of view is a little narrower than that in the aforementioned embodiment.

In the present embodiment, as described above, the wide-angle objective optical system has a wider angle of view, can correct aberrations appropriately, is suitable even for high-definition and high-pixel solid state imaging elements, and can reduce the diameter. The number of the lenses included in the wide-angle objective optical system can be reduced so that the overall length can be decreased. Also, the manufacturing cost can be reduced.

{Examples}

Next, descriptions will be given of Examples 1 to 9 of the wide-angle objective optical system of the aforementioned embodiment with reference to Fig. 5 to Fig. 22. In lens data shown in the Examples, r is a curvature radius (unit: mm), d is a face space (mm), Ne is a refractive index relative to a line e, and Vd is an Abbe number relative to a line d.

Regarding an aspherical face, "*" is added to its face number in the lens data. A paraxial curvature radius r, a conic constant K, an aspheric coefficient Ai (i = 2, 4, 6, 8, and 10), which are expressed by the following expression, are shown in the aspherical face data. In the following expression, an optical axis direction is z and a direction perpendicular to the optical axis is y.

$$z = (y^2/r)/[1 + \{1 - (1 + K)(y/r)^2\}^{1/2}] + A2y^2 + A4y^4 + A6y^6$$
$$+ A8y^8 + A10y^{10}$$

Example 1

Fig. 5 illustrates a configuration of a wide-angle objective optical system of Example 1 of the present invention. Fig. 6 is a diagram of aberration curves of the wide-angle objective optical system of the present example.

Lens data of the wide-angle objective optical system of Example 1 of the present invention is as follows.

Lens Data

| Face No. | r | d | Ne | Vd |
|---|---|---|---|---|
| Object face | ∞ | 7.34 | 1. | |
| 1 | ∞ | 0.2250 | 1.88815 | 40.76 |
| 2 | 0.5303 | 0.2400 | 1. | |
| 3 | -3.2898 | 0.2250 | 1.64116 | 23.90 |
| 4 | -22.5660 | 0.2217 | 1, | |
| 5 (Brightness diaphragm) | ∞ | 0.0375 | 1. | |
| 6 | 1.5164 | 0.1967 | 1.53296 | 55.69 |
| 7 | -1.2232 | 0.9242 | 1. | |
| 8 | 1.3092 | 0.7052 | 1.53296 | 55.69 |
| 9* | -1.2062 | 0.1500 | 1. | |
| 10 | -1.1260 | 0.2272 | 1.64116 | 23.90 |
| 11 | 2.0776 | 0.0375 | 1. | |

(continued)

| Face No. | r | d | Ne | Vd |
|---|---|---|---|---|
| 12 | 1.7475 | 0.8718 | 1.53296 | 55.69 |
| 13* | -2.5172 | 0.9259 | 1. | |
| 14 | ∞ | 0.5506 | 1.51825 | 64.14 |
| 15 | ∞ | 0.0918 | 1. | |
| Image plane | ∞ | | | |

Aspherical face data

9th face

r=-1.2062
K=-0.3682
A2=0.0000E+00
A4=2.5578E-01
A6=1.8699E-01
A8=-4.0304E-01

13th face

r=-2.5172
K=-0.7009
A2=0.0000E+00
A4=-5.6576E-02
A6=3.1115E-02
A8=3.5770E-02
A10=-7.2866E-05

Other data

Focal distance 1.0
Image height 1.01
Fno. 7.03
Effective Fno. 7.15
Objective distance 7.34
Half angle of view 68.5°
Distortion aberration -59.6%

The wide-angle objective optical system of the present example, which has an outer diameter of an end that is substantially same as the image height, is compact. Further, the wide-angle objective optical system achieves an angle of view of 137°. The lenses other than the first lens of the first lens group are adapted to characteristics of existing plastic optical materials with which injection molding can be performed. Thus, the manufacturing cost can be reduced to be low. Moreover, optical performance can be corrected preferably so that the wide-angle objective optical system is applicable to high-definition and high-pixel image pickup elements.

Example 2

Fig. 7 illustrates a configuration of a wide-angle objective optical system of Example 2 of the present invention. Fig. 8 is a diagram of aberration curves of the wide-angle objective optical system of the present example.

Lens data of the wide-angle objective optical system of Example 2 of the present invention is as follows.

Lens Data

| Face No. | r | d | Ne | Vd |
|---|---|---|---|---|
| Object face | ∞ | 7.57 | 1. | |
| 1 | ∞ | 0.2222 | 1.80811 | 46.57 |
| 2 | 0.4927 | 0.2475 | 1. | |
| 3 | -3.4692 | 0.2168 | 1.53296 | 55.69 |
| 4 | -7.8905 | 0.2535 | 1. | |
| 5 (Brightness diaphragm) | ∞ | 0.0387 | 1. | |
| 6 | 1.5923 | 0.4206 | 1.53296 | 55.69 |
| 7 | -1.2446 | 0.8759 | 1. | |
| 8 | 1.3493 | 0.8307 | 1.53296 | 55.69 |
| 9* | -1.3500 | 0.1547 | 1. | |
| 10 | -1.1851 | 0.2089 | 1.64116 | 23.90 |
| 11* | 2.1985 | 0.0387 | 1. | |
| 12 | 1.9569 | 1.3150 | 1.53296 | 55.69 |
| 13* | -2.4476 | 0.4477 | 1. | |
| 14 | ∞ | 0.5415 | 1.51825 | 64.14 |
| 15 | ∞ | 0.0947 | 1. | |
| Image plane | ∞ | | | |

Aspherical face data

9th face

r=-1.3500
K=-0.3596
A2=0.0000E+00
A4=2.0711E-01
A6=1.8713E-01
A8=-2.5548E-01

11th face

r=2.1985
K=-0.0656
A2=0.0000E+00
A4=8.8749E-03
A6=1.8169E-03
A8=4.5301E-04

13th face

r=-2.4476
K=-0.6886

A2=0.0000E+00
A4=-7.6842E-02
A6=6.5255E-03
A8=1.2701E-02
A10=2.1695E-03

Other data

Focal distance 1.0
Image height 1.04
Fno. 7.03
Effective Fno. 7.12
Objective distance 7.57
Half angle of view 80.4°
Distortion aberration -81.8%

The wide-angle objective optical system of the present example, which has an outer diameter of an end that is substantially same as the image height, is compact. Further, the wide-angle objective optical system achieves a very wide angle of view of 160.8°. The lenses other than the first lens of the first lens group can be formed of a material adapted to characteristics of existing plastic optical materials with which injection molding can be performed. Thus, the manufacturing cost can be reduced to be low. Moreover, optical performance can be corrected preferably so that the wide-angle objective optical system is applicable to high-definition and high-pixel image pickup elements.

Example 3

Fig. 9 illustrates a configuration of a wide-angle objective optical system of Example 3 of the present invention. Fig. 10 is a diagram of aberration curves of the wide-angle objective optical system of the present example.

Lens data of the wide-angle objective optical system of Example 3 of the present invention is as follows.

Lens Data

| Face No. | r | d | Ne | Vd |
|---|---|---|---|---|
| Object face | ∞ | 7.71 | 1. | |
| 1 | ∞ | 0.2363 | 1.88815 | 40.76 |
| 2 | 0.5431 | 0.2520 | 1. | |
| 3 | -3.1744 | 0.2207 | 1.53296 | 55.69 |
| 4 | -10.0626 | 0.2574 | 1. | |
| 5 (Brightness diaphragm) | ∞ | 0.0394 | 1. | |
| 6 | 1.6617 | 0.4112 | 1.53296 | 55.69 |
| 7 | -1.2271 | 0.8888 | 1. | |
| 8 | 1.3585 | 0.9015 | 1.53296 | 55.69 |
| 9* | -1.3881 | 0.1575 | 1. | |
| 10 | -1.2238 | 0.2126 | 1.64116 | 23.90 |
| 11* | 2.2871 | 0.0394 | 1. | |
| 12 | 2.0062 | 1.3388 | 1.53296 | 55.69 |
| 13* | -2.5256 | 0.4396 | 1. | |
| 14 | ∞ | 0.5513 | 1.51825 | 64.14 |
| 15 | ∞ | 0.0964 | 1. | |

(continued)

| Face No. | r | d | Ne | Vd |
|---|---|---|---|---|
| Image plane | ∞ | | | |

Aspherical face data

9th face

r=-1.3881
K=-0.3653
A2=0.0000E+00
A4=2.1119E-01
A6=1.7193E-01
A8=-2.1072E-01

11th face

r=2.2871
K=-0.0522
A2=0.0000E+00
A4=2.1972E-03
A6=1.5395E-03
A8=-2.4528E-03

13th face

r=-2.5256
K=-0.6925
A2=0.0000E+00
A4=-6.7290E-02
A6=8.1438E-03
A8=1.6600E-02
A10=-7.1607E-04

Other data

Focal distance 1.0
Image height 1.06
Fno. 6.86
Effective Fno. 6.94
Objective distance 7.71
Half angle of view 82.0°
Distortion aberration -84.6%

The wide-angle objective optical system of the present example, which has an outer diameter of an end that is substantially same as the image height, is compact. Further, the wide-angle objective optical system achieves a very wide angle of view of 164.0°. The lenses other than the first lens of the first lens group can be formed of a material adapted to characteristics of existing plastic optical materials with which injection molding can be performed. Thus, the manufacturing cost can be reduced to be low. Moreover, optical performance can be corrected preferably so that the wide-angle objective optical system is applicable to high-definition and high-pixel image pickup elements.

Example 4

Fig. 11 illustrates a configuration of a wide-angle objective optical system of Example 4 of the present invention. Fig. 12 is a diagram of aberration curves of the wide-angle objective optical system of the present example.

Lens data of the wide-angle objective optical system of Example 4 of the present invention is as follows.

Lens Data

| Face No. | r | d | Ne | Vd |
|---|---|---|---|---|
| Object face | ∞ | 7.26 | 1. | |
| 1 | ∞ | 0.2214 | 1.88815 | 40.76 |
| 2 | 0.5302 | 0.2362 | 1. | |
| 3 | -3.4490 | 0.2214 | 1.64116 | 23.90 |
| 4 | -13.2109 | 0.2315 | 1. | |
| 5 (Brightness diaphragm) | ∞ | 0.0369 | 1. | |
| 6 | 1.3615 | 0.2145 | 1.53296 | 55.69 |
| 7 | -1.2179 | 0.9486 | 1. | |
| 8 | 1.2790 | 0.6072 | 1.53296 | 55.69 |
| 9* | -1.3060 | 0.1476 | 1. | |
| 10 | -1.4980 | 0.2527 | 1.85504 | 23.78 |
| 11 | 2.8365 | 0.0369 | 1. | |
| 12 | 1.9192 | 0.8580 | 1.53296 | 55.69 |
| 13* | -2.3746 | 0.8169 | 1. | |
| 14 | ∞ | 0.5419 | 1.51825 | 64.14 |
| 15 | ∞ | 0.0903 | 1. | |
| Image plane | ∞ | | | |

Aspherical face data

9th face

r=-1.3060
K=-0.3864
A2=0.0000E+00
A4=3.1029E-01
A6=2.1928E-01
A8=-3.2078E-01

13th face

r=-2.3746
K=-0.6988
A2=0.0000E+00
A4=-6.1785E-02
A6=3.8835E-03
A8=3.8809E-02
A10=-1.3094E-03

Other data

Focal distance 1.0
Image height 0.993
Fno. 7.12
Effective Fno. 7.20
Objective distance 7.26
Half angle of view 69.0°
Distortion aberration -61.4%

The wide-angle objective optical system of the present example, which has an outer diameter of an end that is substantially same as the image height, is compact. Further, the wide-angle objective optical system achieves a very wide angle of view of 164.0°. The lenses other than the first lens of the first lens group and the second lens of the second group can be formed of a material adapted to characteristics of existing plastic optical materials with which injection molding can be performed. Thus, the manufacturing cost can be reduced to be low. Moreover, optical performance can be corrected preferably so that the wide-angle objective optical system is applicable to high-definition and high-pixel image pickup elements.

Example 5

Fig. 13 illustrates a configuration of a wide-angle objective optical system of Example 5 of the present invention. Fig. 14 is a diagram of aberration curves of the wide-angle objective optical system of the present example.

Lens data of the wide-angle objective optical system of Example 5 of the present invention is as follows.

Lens Data

| Face No. | r | d | Ne | Vd |
|---|---|---|---|---|
| Object face | ∞ | 8.18 | 1. | |
| 1 | ∞ | 0.2505 | 1.88815 | 40.76 |
| 2 | 0.6109 | 0.2672 | 1. | |
| 3 | -4.3397 | 0.2340 | 1.53296 | 55.69 |
| 4 | -241.7170 | 0.3217 | 1. | |
| 5 (Brightness diaphragm) | ∞ | 0.0418 | 1. | |
| 6 | 2.3344 | 0.3621 | 1.53296 | 55.69 |
| 7 | -1.3748 | 0.8690 | 1. | |
| 8 | 1.4063 | 0.9028 | 1.53296 | 55.69 |
| 9* | -1.4833 | 0.1670 | 1. | |
| 10 | -1.2593 | 0.2255 | 1.64116 | 23.90 |
| 11* | 2.3544 | 0.1086 | 1. | |
| 12 | 4.1849 | 0.8351 | 1.53296 | 55.69 |
| 13 | -3.3589 | 0.0418 | 1. | |
| 14 | 3.3680 | 1.0022 | 1.53296 | 55.69 |
| 15* | -4.1540 | 0.5489 | 1. | |
| 16 | ∞ | 0.5846 | 1.51825 | 64.14 |
| 17 | ∞ | 0.1022 | 1. | |
| Image plane | ∞ | | | |

Aspherical face data

9th face

r=-1.4833
K=-0.3350
A2=0.0000E+00
A4=1.4162E-01
A6=9.2723E-02
A8=-1.6871E-01
11th face
r=2.3544
K= -0.1943
A2=0.0000E+00
A4=6.2544E-03
A6=2.7560E-03
A8=4.5632E-03
15th face
r=-4.1540
K=-0.6724
A2=0.0000E+00
A4=-3.1750E-03
A6=-1.3999E-03
A8=1.1768E-03
A10=9.6393E-04

Other data

Focal distance 1.0
Image height 1.12
Fno. 6.51
Effective Fno. 6.50
Objective distance 8.18
Half angle of view 82.0°
Distortion aberration -83.4%

The wide-angle objective optical system of the present example, which has an outer diameter of an end that is less than 1.2 times of the image height, is compact. Further, the wide-angle objective optical system achieves a very wide angle of view of 164.0°. The lenses other than the first lens of the first group can be formed of a material adapted to characteristics of existing plastic optical materials with which injection molding can be performed. Thus, the manufacturing cost can be reduced to be low. Moreover, optical performance can be corrected preferably so that the wide-angle objective optical system is applicable to high-definition and high-pixel image pickup elements.

Example 6

Fig. 15 illustrates a configuration of a wide-angle objective optical system of Example 6 of the present invention. Fig. 16 is a diagram of aberration curves of the wide-angle objective optical system of the present example.

Lens data of the wide-angle objective optical system of Example 6 of the present invention is as follows.

Lens Data

| Face No. | r | d | Ne | Vd |
|---|---|---|---|---|
| Object face | ∞ | 8.04 | 1. | |

(continued)

| Face No. | r | d | Ne | Vd |
|---|---|---|---|---|
| 1 | ∞ | 0.2359 | 1.80811 | 46.57 |
| 2 | 0.5554 | 0.2628 | 1. | |
| 3 | -4.7506 | 0.2301 | 1.53296 | 55.69 |
| 4 | -14.7055 | 0.3138 | 1. | |
| 5 (Brightness diaphragm) | ∞ | 0.0411 | 1. | |
| 6 | 2.3493 | 0.3634 | 1.53296 | 55.69 |
| 7 | -1.3844 | 0.8988 | 1. | |
| 8 | 1.3918 | 0.8675 | 1.53296 | 55.69 |
| 9* | -1.4475 | 0.1642 | 1. | |
| 10 | -1.2200 | 0.2217 | 1.64116 | 23.90 |
| 11* | 2.2838 | 0.1067 | 1. | |
| 12 | 3.9162 | 0.8211 | 1.53296 | 55.69 |
| 13* | -3.2321 | 0.0411 | 1. | |
| 14 | 3.3038 | 0.9853 | 1.53296 | 55.69 |
| 15* | -4.0534 | 0.5094 | 1. | |
| 16 | ∞ | 0.5748 | 1.51825 | 64.14 |
| 17 | ∞ | 0.1005 | 1. | |
| Image plane | ∞ | | | |

Aspherical face data

9th face

r=-1.4475
K=-0.3267
A2=0.0000E+00
A4=1.3199E-01
A6=1.2211E-01
A8=-2.0728E-01

11th face

r=2.2838
K=-0.1994
A2=0.0000E+00
A4=5.7995E-04
A6=-1.1190E-04
A8=9.0082E-03

13th face

r=-3.2321
K=0.0000

A2=0.0000E+00
A4=1.0619E-03
A6=-8.9082E-04
A8=2.4576E-05
A10=9.9123E-04

15th face

r=-4.0534
K=-0.6719
A2=0.0000E+00
A4=-7.4185E-03
A6=-7.6408E-04
A8=1.8193E-03
A10=4.1008E-04

Other data

Focal distance 1.0
Image height 1.11
Fno. 7.09
Effective Fno. 7.06
Objective distance 8.04
Half angle of view 80.1°
Distortion aberration -79.7%

The wide-angle objective optical system of the present example, which has an outer diameter of an end that is less than 1.2 times of the image height, is compact. Further, the wide-angle objective optical system achieves a very wide angle of view of 160.2°. The lenses other than the first lens of the first group can be formed of a material adapted to characteristics of existing plastic optical materials with which injection molding can be performed. Thus, the manufacturing cost can be reduced to be low. Moreover, optical performance can be corrected preferably so that the wide-angle objective optical system is applicable to high-definition and high-pixel image pickup elements.

Example 7

Fig. 17 illustrates a configuration of a wide-angle objective optical system of Example 7 of the present invention. Fig. 18 is a diagram of aberration curves of the wide-angle objective optical system of the present example.

Lens data of the wide-angle objective optical system of Example 7 of the present invention is as follows.

Lens Data

| Face No. | r | d | Ne | Vd |
|---|---|---|---|---|
| Object face | ∞ | 7.44 | 1. | |
| 1 | ∞ | 0.2184 | 1.80811 | 46.57 |
| 2 | 0.4889 | 0.2433 | 1. | |
| 3 | -4.4397 | 0.2130 | 1.53296 | 55.69 |
| 4 | -4.7905 | 0.2596 | 1. | |
| 5 (Brightness diaphragm) | ∞ | 0.0380 | 1. | |
| 6 | 1.9357 | 0.4322 | 1.53296 | 55.69 |
| 7 | -1.1697 | 0.8118 | 1. | |
| 8 | 1.3220 | 0.8349 | 1.53296 | 55.69 |

(continued)

| Face No. | r | d | Ne | Vd |
|---|---|---|---|---|
| 9* | -1.3119 | 0.1520 | 1. | |
| 10 | -1.1751 | 0.2053 | 1.64116 | 23.90 |
| 11 | 1.8701 | 1.2924 | 1.53296 | 55.69 |
| 12* | -2.3764 | 0.4228 | 1. | |
| 13 | ∞ | 0.5322 | 1.51825 | 64.14 |
| 14 | ∞ | 0.0930 | 1. | |
| Image plane | ∞ | | | |

Aspherical face data

9th face

r=-1.3119
K=-0.3507
A2=0.0000E+00
A4=2.2698E-01
A6=1.7326E-01
A8=-2.8028E-01

12th face

r=-2.3764
K=-0.6737
A2=0.0000E+00
A4=-8.7267E-02
A6=1.1711E-02
A8=3.3688E-02
A10=-1.3511E-02

Other data

Focal distance 1.0
Image height 1.02
Fno. 6.91
Effective Fno. 7.20
Objective distance 7.44
Half angle of view 70.3°
Distortion aberration -62.7%

The wide-angle objective optical system of the present example, which has an outer diameter of an end that is substantially same as the image height, is compact. Further, the wide-angle objective optical system achieves an angle of view of 140.6°. The lenses other than the first lens of the first lens group are adapted to characteristics of existing plastic optical materials with which injection molding can be performed. Thus, the manufacturing cost can be reduced to be low. Moreover, optical performance can be corrected preferably so that the wide-angle objective optical system is applicable to high-definition and high-pixel image pickup elements.

# EP 2 990 847 A1

Example 8

Fig. 19 illustrates a configuration of a wide-angle objective optical system of Example 8 of the present invention. Fig. 20 is a diagram of aberration curves of the wide-angle objective optical system of the present example.

Lens data of the wide-angle objective optical system of Example 8 of the present invention is as follows.

Lens Data

| Face No. | r | d | Ne | Vd |
|---|---|---|---|---|
| Object face | ∞ | 6.71 | 1. | |
| 1 | ∞ | 0.2058 | 1.53296 | 55.69 |
| 2 | ∞ | 0.0420 | 1. | |
| 3 | 10.6871 | 0.2058 | 1.53296 | 55.69 |
| 4* | 0.4072 | 0.2404 | 1. | |
| 5 | -2.8467 | 0.2058 | 1.53296 | 55.69 |
| 6 | -8.2032 | 0.1935 | 1. | |
| 7 (Brightness diaphragm) | ∞ | 0.0343 | 1. | |
| 8 | 1.4544 | 0.2047 | 1.53296 | 55.69 |
| 9 | -1.5617 | 0.6498 | 1. | |
| 10 | 1.2805 | 0.7545 | 1.53296 | 55.69 |
| 11* | -0.9639 | 0.1372 | 1. | |
| 12 | -1.1255 | 0.2744 | 1.64116 | 23.90 |
| 13 | 1.8786 | 0.0343 | 1. | |
| 14 | 1.6322 | 1.0289 | 1.53296 | 55.69 |
| 15* | -1.9349 | 0.3990 | 1. | |
| 16 | ∞ | 0.5036 | 1.51825 | 64.14 |
| 17 | ∞ | 0.0839 | 1. | |
| Image plane | ∞ | | | |

Aspherical face data

4th face

```
r=0.4072
K=-0.5908
A2=0.0000E+00
A4=1.4061E+00
A6=3.3904E-01
A8=-1.8113E+00
```

11th face

```
r=-0.9639
K=-0.3711
A2=0.0000E+00
A4=3.4673E-01
```

A6=7.5552E-01
A8=-1.2819E+00

15th face

r=-1.9349
K=-0.7593
A2=0.0000E+00
A4=1.7180E-03
A6=-1.8526E-01
A8=2.5126E-O1
A10=-1.9675E-02

Other data

Focal distance 1.0
Image height 0.923
Fno. 6.83
EffectiveFno. 6.93
Objective distance 6.71
Half angle of view 51.9°
Distortion aberration -27.0%

The wide-angle objective optical system of the present example, which has an outer diameter of a parallel plate for protection of an end that is 1.5 times of the image height, is compact. Further, the wide-angle objective optical system achieves a wide angle of view of 103.8°. All lenses can be formed of a material adapted to characteristics of existing plastic optical materials with which injection molding can be performed. Thus, the manufacturing cost can be reduced to be low. Moreover, optical performance can be corrected preferably so that the wide-angle objective optical system is applicable to high-definition and high-pixel image pickup elements. The parallel plate for protection may be formed of optical glass, which is resistant to damage, instead of a plastic optical material.

Example 9

Fig. 20 illustrates a configuration of a wide-angle objective optical system of Example 9 of the present invention. Fig. 21 is a diagram of aberration curves of the wide-angle objective optical system of the present example.
Lens data of the wide-angle objective optical system of Example 9 of the present invention is as follows.

{Table 25}

| Lens Data | | | | |
|---|---|---|---|---|
| Face No. | r | d | Ne | Vd |
| Object face | ∞ | 6.62 | 1. | |
| 1 | ∞ | 0.2029 | 1.53296 | 55.69 |
| 2 | ∞ | 0.0415 | 1. | |
| 3 | 11.9388 | 0.2029 | 1.53296 | 55.69 |
| 4* | 0.3999 | 0.2384 | 1. | |
| 5 | -2.7645 | 0.2029 | 1.53296 | 55.69 |
| 6 | -8.2657 | 0.1932 | 1. | |
| STO | ∞ | 0.0338 | 1. | |
| 8 | 1.3707 | 0.2038 | 1.53296 | 55.69 |
| 9 | -1.5017 | 0.6234 | 1. | |

(continued)

| Lens Data | | | | |
|---|---|---|---|---|
| Face No. | r | d | Ne | Vd |
| 10 | 1.2580 | 0.7441 | 1.53296 | 55.69 |
| 11* | -0.9491 | 0.1353 | 1. | |
| 12* | -1.1132 | 0.2706 | 1.64116 | 23.90 |
| 13 | 1.5998 | 1.0147 | 1.53296 | 55.69 |
| 14* | -1.8606 | 0.3854 | 1. | |
| 15 | ∞ | 0.4966 | 1.51825 | 64.14 |
| 16 | ∞ | 0.0828 | 1. | |
| Image plane | ∞ | | | |

Aspherical face data

4th face

r=0.3999
K=-0.6259
A2=0.0000E+00
A4=1.1956E+00
A6=1.4816E+00
A8=7.0716E+00

11th face

r=-0.9491
K=-0.3856
A2=0.0000E+00
A4=4.1595E-01
A6=7.1883E-01
A8=-1.2321E+00

12th face

r=-1.1132
K=0.0303
A2=0.0000E+00
A4=-3.6151E-05
A6=4.5121E-05
A8=-1.5651E-03

14th face

r=-1.8606
K=-0.7525
A2=0.0000E+00
A4=5.4669E-03
A6=-2.4071E-01
A8=2.0911E-01
A10=3.2784E-02

Other data

Focal distance 1.0
Image height 0.910
Fno. 6.97
EffectiveFno. 7.11
Objective distance 6.62
Half angle of view 58.3°
Distortion aberration -24.3%

The wide-angle objective optical system of the present example, which has an outer diameter of a parallel plate for protection of an end that is less than 1.5 times of the image height, is compact. Further, the wide-angle objective optical system achieves a wide angle of view of 116.6°. All lenses can be formed of a material adapted to characteristics of existing plastic optical materials with which injection molding can be performed. Thus, the manufacturing cost can be reduced to be low. Moreover, optical performance can be corrected preferably so that the wide-angle objective optical system is applicable to high-definition and high-pixel image pickup elements. The parallel plate for protection may be formed of optical glass, which is resistant to damage, instead of a plastic optical material.

Numerical values of the condition expressions (1) to (5) in the configurations of the aforementioned Examples 1 to 9 are shown in Table 1.

TABLE. 1

| CONDITION EXPRESSION | EXAMPLE 1 | EXAMPLE 2 | EXAMPLE 3 | EXAMPLE 4 | EXAMPLE 5 | EXAMPLE 6 | EXAMPLE 7 | EXAMPLE 8 | EXAMPLE 9 |
|---|---|---|---|---|---|---|---|---|---|
| (10)f/f1 | -0,135 | 0.0374 | 0.0112 | -0.0060 | -0.148 | -0.124 | 0.0635 | -0.0797 | -0.0405 |
| (2)f/f2 | 0.456 | 0.401 | 0.398 | 0.437 | 0.406 | 0.415 | 0.398 | 0.536 | 0.527 |
| (3)D/f | 0.924 | 0.876 | 0.889 | 0.949 | 0.869 | 0.899 | 0.812 | 0.650 | 0.623 |
| (4)f/f13 | 0.768 | 0.724 | 0.718 | 0.805 | 0.595 | 0.591 | 0.696 | 0.691 | 0.725 |
| (5)f/f12 | -0.166 | -0.0846 | -0.114 | -0.136 | -0.121 | -0.0753 | -0.0069 | -0.121 | -0.127 |

{Reference Signs List}

| 1 | Wide-angle objective optical system |
|---|---|
| G1 | First lens group |
| G2 | Second lens group |
| L1 | First lens |
| L2 | Second lens |
| L3 | Third lens |
| L4 | Fourth lens |
| L5 | Fifth lens |
| L6 | Sixth lens |
| L7 | Seventh lens |
| CL1 | Cemented lens |
| S | Brightness diaphragm |
| F | Parallel plate |

**Claims**

1. A wide-angle objective optical system comprising, in order from an object side to an image side:

   a first lens group; and
   a second lens group having positive refractive power,

   wherein the first lens group comprises, in order from the object side to the image side:

   a first lens having negative refractive power;
   a second lens having a concave face as an object-side face and having negative refractive power;
   a brightness diaphragm; and
   a third lens having positive refractive power, and following condition expressions are satisfied:

$$-0.5 < f/f1 < 0.5 \qquad (1)$$

$$0.3 < f/f2 < 0.7 \qquad (2)$$

   wherein f1 is a focal distance of the first lens group; f2 is a focal distance of the second lens group; and f is a focal distance of the entire wide-angle objective optical system.

2. The wide-angle objective optical system of Claim 1, wherein a following condition expression is satisfied:

$$0.8 < D/f < 1.2 \qquad (3)$$

   wherein D is an air space between a curved face having refractive power nearest to an image of the first lens group and a curved face having refractive power nearest to an object of the second lens group.

3. The wide-angle objective optical system of Claim 2, wherein the second lens group comprises, in order from the object side to the image side, a fourth lens having positive refractive power, a fifth lens having negative refractive power and a sixth lens having positive refractive power.

4. The wide-angle objective optical system of Claim 2, wherein the second lens group comprises, in order from the object side to the image side, a fourth lens having positive refractive power, a fifth lens having negative refractive power, a sixth lens having positive refractive power and a seventh lens having positive refractive power.

5. The wide-angle objective optical system of any one of Claims 1 to 4, wherein a following condition expression is satisfied:

$$0.5 < f/f13 < 0.9 \qquad (4)$$

wherein f13 is a focal distance of the third lens having positive refractive power of the first lens group.

6. The wide-angle objective optical system of Claim 5, wherein a following condition expression is satisfied:

$$-0.3 < f/f12 < 0.0 \qquad (5)$$

wherein f12 is a focal distance of the second lens having negative refractive power of the first lens group.

TABLE. 1

| CONDITION EXPRESSION | EXAMPLE 1 | EXAMPLE 2 | EXAMPLE 3 | EXAMPLE 4 | EXAMPLE 5 | EXAMPLE 6 | EXAMPLE 7 | EXAMPLE 8 | EXAMPLE 9 |
|---|---|---|---|---|---|---|---|---|---|
| (1)f/f1 | -0.135 | 0.0374 | 0.0112 | -0.0060 | -0.148 | -0.124 | 0.0635 | -0.0797 | -0.0405 |
| (2)f/f2 | 0.456 | 0.401 | 0.398 | 0.437 | 0.406 | 0.415 | 0.398 | 0.536 | 0.527 |
| (3)D/f | 0.924 | 0.876 | 0.889 | 0.949 | 0.869 | 0.899 | 0.812 | 0.650 | 0.623 |
| (4)f/f13 | 0.768 | 0.724 | 0.718 | 0.805 | 0.595 | 0.591 | 0.696 | 0.691 | 0.725 |
| (5)f/f12 | -0.166 | -0.0846 | -0.114 | -0.136 | -0.121 | -0.0753 | -0.0069 | -0.121 | -0.127 |

FIG. 1

EP 2 990 847 A1

FIG. 2

G2

G1p

G1n

G1

FIG. 3

EP 2 990 847 A1

FIG. 4

FIG. 5

# FIG. 6

| SPHERICAL ABERRATION (LONGITUDINAL) | ASTIGMATISM | CHROMATIC ABERRATION OF MAGNIFICATION | DISTORTION ABERRATION (%) | COMA ABERRATION (LONGITUDINAL) |
|---|---|---|---|---|
| FNO 7.029 | FIY 1.01 | FIY 1.01 | FIY 1.01 | 1.109 |

-0.05    0.05

-0.05    0.05

-0.01    0.01

-50.00    50.00

-0.05 ┼┼┼┼┼┼┼┼ 0.05

-1.109

| | | |
|---|---|---|
| 546.07 ——— | SAGITTAL IMAGE PLANE ——— | 546.07 ——— |
| 435.84 ——— | | 435.84 ——— |
| 486.13 ——— | MERIDIONAL IMAGE PLANE ——— | 486.13 ——— |
| 686.28 ——— | | 686.28 ——— |

FIG. 7

# FIG. 8

SPHERICAL ABERRATION (LONGITUDINAL)
FNO 7.029

ASTIGMATISM
FIY 1.04

CHROMATIC ABERRATION OF MAGNIFICATION
FIY 1.04

DISTORTION ABERRATION (%)
FIY 1.04

COMA ABERRATION (LONGITUDINAL)
1.160

-0.05    0.05

-0.05    0.05

-0.01    0.01

-50.00    50.00

-0.05 +++++++ 0.05

-1.160

546.07 ——————
435.84 — — — —
486.13 —·—·—·—
686.28 ------------

SAGITTAL IMAGE PLANE ——————

MERIDIONAL IMAGE PLANE — — —

546.07 ——————
435.84 — — — —
486.13 —·—·—·—
686.28 ------------

EP 2 990 847 A1

# FIG. 9

EP 2 990 847 A1

FIG. 10

# FIG. 11

EP 2 990 847 A1

# FIG. 12

| SPHERICAL ABERRATION (LONGITUDINAL) | ASTIGMATISM | CHROMATIC ABERRATION OF MAGNIFICATION | DISTORTION ABERRATION (%) | COMA ABERRATION (LONGITUDINAL) |
|---|---|---|---|---|
| FNO 7.125 | FIY 0.99 | FIY 0.99 | FIY 0.99 | 1.109 |

-0.05    0.05      -0.05    0.05      -0.01    0.01      -50.00    50.00      -0.05 ├┼┼┼┼┼┼┤ 0.05

-1.109

546.07 ————
435.84 — — —
486.13 —·—·—
686.28 ----------

SAGITTAL IMAGE PLANE ————

MERIDIONAL IMAGE PLANE — — —

435.84 — — —
486.13 —·—·—
686.28 ----------

EP 2 990 847 A1

FIG. 13

# FIG. 14

| SPHERICAL ABERRATION (LONGITUDINAL) | ASTIGMATISM | CHROMATIC ABERRATION OF MAGNIFICATION | DISTORTION ABERRATION (%) | COMA ABERRATION (LONGITUDINAL) |
|---|---|---|---|---|
| FNO 6.512 | FIY 1.12 | FIY 1.12 | FIY 1.12 | 1.132 |

-0.05    0.05
-0.05    0.05
-0.01    0.01
-50.00    50.00
-0.05    0.05
-1.132

546.07 ——
435.84 — — —
486.13 —·—·—
686.28 ----------

SAGITTAL
IMAGE PLANE ——

MERIDIONAL — — —
IMAGE PLANE

435.84 — — —
486.13 —·—·—
686.28 ----------

EP 2 990 847 A1

FIG. 15

# FIG. 16

| SPHERICAL ABERRATION (LONGITUDINAL) FNO 7.088 | ASTIGMATISM FIY 1.11 | CHROMATIC ABERRATION OF MAGNIFICATION FIY 1.11 | DISTORTION ABERRATION (%) FIY 1.11 | COMA ABERRATION (LONGITUDINAL) 1.144 |

-0.05  0.05     -0.05  0.05     -0.01  0.01     -50.00  50.00     -0.05 ⊢⊢⊢⊢⊢ 0.05

1.144
−1.144

546.07 ——————
435.84 —— —— ——
486.13 —— · —— ·
686.28 ----------

SAGITTAL
IMAGE PLANE ——————

MERIDIONAL ____ ____
IMAGE PLANE

435.84 —— —— ——
486.13 —— · —— ·
686.28 ----------

EP 2 990 847 A1

FIG. 17

# FIG. 18

SPHERICAL ABERRATION
(LONGITUDINAL)
FNO 6.909

ASTIGMATISM
FIY 1.02

CHROMATIC ABERRATION
OF MAGNIFICATION
FIY 1.02

DISTORTION
ABERRATION (%)
FIY 1.02

COMA ABERRATION
(LONGITUDINAL)
1.133

-0.05 — 0.05

-0.05        0.05

-0.05        0.05

-0.01        0.01

-50.00       50.00

-1.133

546.07 ————
435.84 ————
486.13 —·—·—
686.28 ----------

SAGITTAL
IMAGE PLANE ————

MERIDIONAL
IMAGE PLANE ————

435.84 ————
486.13 —·—·—
686.28 ----------

EP 2 990 847 A1

FIG. 19

# FIG. 20

SPHERICAL ABERRATION (LONGITUDINAL) FNO 6.827 / ASTIGMATISM FIY 0.92 / CHROMATIC ABERRATION OF MAGNIFICATION FIY 0.92 / DISTORTION ABERRATION (%) FIY 0.92 / COMA ABERRATION (LONGITUDINAL) 1.204

EP 2 990 847 A1

# FIG. 21

# FIG. 22

SPHERICAL ABERRATION
(LONGITUDINAL)
FNO 6.973

ASTIGMATISM
FIY 0.91

CHROMATIC ABERRATION
OF MAGNIFICATION
FIY 0.91

DISTORTION
ABERRATION (%)
FIY 0.91

COMA ABERRATION
(LONGITUDINAL)
1.185

-0.05            0.05

-0.05            0.05

-0.01            0.01

-50.00          50.00

-0.05 ++++++++ 0.05

-1.185

546.07 ——————
435.84 —— —— ——
486.13 ——·——·——
686.28 ------------

SAGITTAL
IMAGE PLANE ——————

MERIDIONAL ____ ____
IMAGE PLANE

435.84 —— —— ——
486.13 ——·——·——
686.28 ------------

EP 2 990 847 A1

## INTERNATIONAL SEARCH REPORT

| | International application No. |
|---|---|
| | PCT/JP2014/059896 |

A. CLASSIFICATION OF SUBJECT MATTER
*G02B13/04*(2006.01)i, *A61B1/00*(2006.01)i, *G02B13/18*(2006.01)i

According to International Patent Classification (IPC) or to both national classification and IPC

B. FIELDS SEARCHED

Minimum documentation searched (classification system followed by classification symbols)
G02B13/04, A61B1/00, G02B13/18

Documentation searched other than minimum documentation to the extent that such documents are included in the fields searched
Jitsuyo Shinan Koho        1922-1996    Jitsuyo Shinan Toroku Koho    1996-2014
Kokai Jitsuyo Shinan Koho    1971-2014    Toroku Jitsuyo Shinan Koho    1994-2014

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2006-171597 A (Matsushita Electric Industrial Co., Ltd.), 29 June 2006 (29.06.2006), examples 1 to 7; paragraph [0029] (Family: none) | 1,3,5 |
| X | JP 2007-279632 A (Matsushita Electric Industrial Co., Ltd.), 25 October 2007 (25.10.2007), carrying-out modes 2 to 8 (Family: none) | 1-3,5 |
| X | JP 2006-349920 A (Ricoh Co., Ltd.), 28 December 2006 (28.12.2006), examples 2, 3 & EP 1734393 A1 | 1-3,6 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 25 June, 2014 (25.06.14) | 08 July, 2014 (08.07.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2014/059896

C (Continuation). DOCUMENTS CONSIDERED TO BE RELEVANT

| Category* | Citation of document, with indication, where appropriate, of the relevant passages | Relevant to claim No. |
|---|---|---|
| X | JP 4-267212 A (Copal Co., Ltd.), 22 September 1992 (22.09.1992), 2nd example (Family: none) | 1-3 |
| X | JP 2011-017900 A (Fujifilm Corp.), 27 January 2011 (27.01.2011), examples 1, 4, 5, 7 to 15 & US 2011/0007401 A1 & CN 201773216 U | 1-2,4-6 |
| X | JP 2000-019391 A (Konica Corp.), 21 January 2000 (21.01.2000), examples 3, 4 (Family: none) | 1-2,4-5 |
| X | JP 2010-085484 A (Kyocera Optec Co., Ltd.), 15 April 2010 (15.04.2010), examples 1 to 6 (Family: none) | 1-2,4 |
| X | JP 2004-177435 A (Ricoh Co., Ltd.), 24 June 2004 (24.06.2004), 2nd example (Family: none) | 1,3,5 |
| X | JP 2001-133685 A (Matsushita Electric Industrial Co., Ltd.), 18 May 2001 (18.05.2001), carrying-out mode 3 (Family: none) | 1,4-5 |
| X | JP 2006-513449 A (3M Innovative Properties Co.), 20 April 2006 (20.04.2006), examples 1 to 3 & US 2004/0130799 A1 & EP 1581831 A & WO 2004/063787 A1 | 1,4,6 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI1020189 B
- JP HEI10197787 B
- JP SHO61162021 B